# EUROPEAN PATENT SPECIFICATION

(11) **EP 1 566 191 B1**
(45) Date of publication and mention of the grant of the patent: **25.04.2012**
(21) Application number: 05003022.0
(22) Date of filing: 14.02.2005
(51) Int. Cl.: A61M 1/36, A61M 1/02

(54) **Method for separating a volume of composite liquid into at least two components**
Verfahren zum Trennen von einem Flüssigkeitsgemischvolumen in wenigstens zwei Komponenten
Procédé pour séparer un volume d'un liquide composite en au moins deux composants

(30) Priority: 20.02.2004 US 546637 P
(43) Date of publication of application: 24.08.2005
(60) Divisional application: 10003381.0
(73) Proprietor: CaridianBCT, Inc., Lakewood, CO 80215 (US)
(72) Inventor: Holmes, Brian M., Lakewood Colorado 80227 (US); van Waeg, Geert, 1200 Brussels (BE); Pihlstedt, Peter, 11422 Stockholm (SE)
(74) Representative: Roberts, Mark Peter

(56) References cited:
- WO-A-00/54823
- WO-A-03/089027
- WO-A-03/106040
- WO-A-2004/018021
- DE-A1- 19 728 089
- US-A- 5 733 545

## Description

The present invention relates to a method for separating a volume of composite liquid into at least two components.

The method of the invention is particularly appropriate for the separation of biological fluids comprising an aqueous component and one or more cellular components. For example, potential uses of the invention include: extracting a plasma component and a red blood cell component from a volume of filtered blood obtained by flowing of a volume of whole blood through a filter removing platelets and white blood cells therefrom; extracting a plasma component, a platelet component and a red blood cell component from a volume of filtered blood obtained by flowing a volume of whole blood through a filter removing white blood cells therefrom; extracting a plasma component and a cellular component (including platelets, white blood cells, and red blood cells) from a volume of whole blood, the cellular component being subsequently filtered so as to remove platelets and white blood cells therefrom; extracting a plasma component, a platelet component, and a red blood cell component from a volume of whole blood, the white blood cells being subsequently removed by filtration from the platelet component and the red blood cell component.

An apparatus for processing blood components is known from document WO 03/089027. This apparatus comprises a centrifuge adapted to cooperate with an annular separation bag connected to at least one product bag, e.g. a platelet component bag. The centrifuge includes:
- a rotor having a turntable for supporting the separation bag, and a central compartment for containing the product bag connected to the separation bag; and
- a squeezing system for squeezing the separation bag and causing the transfer of a separated component (e.g. platelets suspended in a diluting solution) from the separation bag into the product bag.

One aim of the present invention is to provide an optimized separation process for separating, in a minimum amount of time, a composite fluid, such as whole blood, into at least two high quality components.

The invention is defined in the claims.

Other features and advantages of the invention will appear from the following description and accompanying drawings, which are to be considered exemplary only.

In the accompanying drawings:
Figure 1 is a schematic view of set of separation and collection bags designed for cooperating with a separation apparatus used for the invention;
Figure 2 is a top plan view of a separation bag designed for cooperating with a separation apparatus used for the invention;
Figure 3 is a schematic view, partly in cross-section, of a separation apparatus used for the invention;
Figure 4 is a cross-section view of the rotor of a separation apparatus used for the invention; and
Figure 5 is a perspective view of the upper part of the rotor of the separation apparatus of figure 4.

For the sake of clarity, the invention will be described with respect to a specific use, namely the separation of whole blood into at least two components, in particular into a first component comprising plasma, a second component comprising platelets and a third component comprising red blood cells. It should be understood however that this specific use is exemplary only.

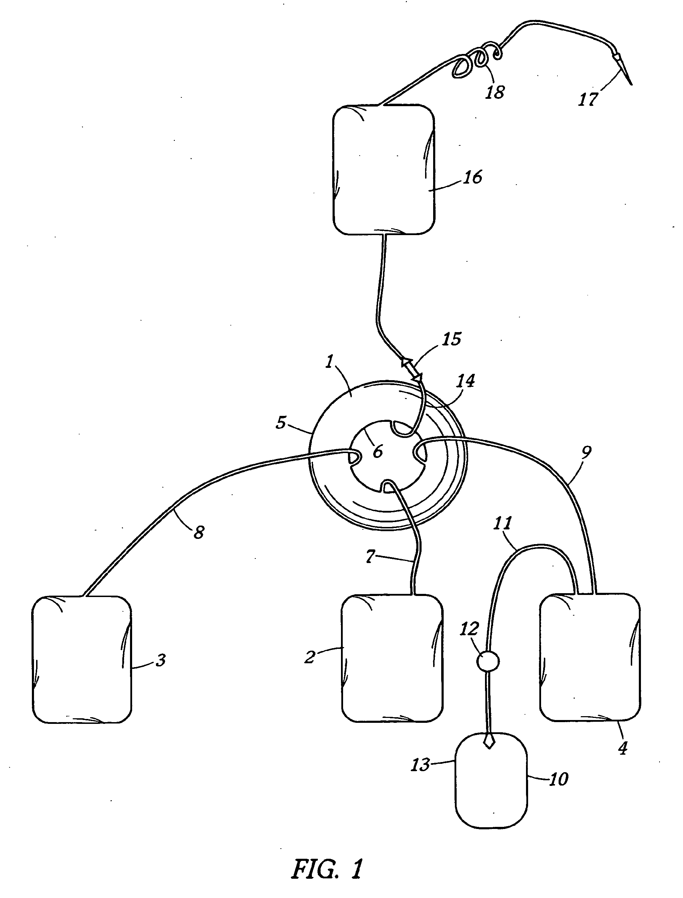

A set of separation bags adapted to the separation of whole blood into a plasma product, a platelet product and red blood cell product is shown in figure 1. This set comprises a separation bag 1 and three product bags 2, 3, 4. The separation bag 1 is annular and has an outer circular edge 5 and an inner circular edge 6. Variants of the separation bag include one or two radial walls extending from the inner edge 6 to the outer edge 5 so that the chamber defined within the bag, instead of being annular, has a C-shape with the C being more or less open. Also the separation bag can be shaped so as to fit either on a flat support surface or on a frusto-conical support surface of the rotor of a centrifuge. The first product bag 2, intended for containing the plasma product, is connected by a first tube 7 to the separation bag 1, at the inner edge 6 thereof. The second product bag 3, intended for containing the platelet product, is connected by a second tube 8 to the separation bag 1, at the inner edge 6 thereof. The third product bag 4, intended for containing the red blood cell product, is connected by a third tube 9 to the separation bag 1, at the inner edge 6 thereof. It is connected to a secondary bag 10 by a tube 11 having two segments respectively connected to the inlet and the outlet of a leukoreduction filter 12 (a filter for removing white blood cells). The secondary bag 10 contains a volume of storage solution for red blood cells. A plug 13 removable from within the secondary bag 10 (so-called "frangible pin", for example) blocks a liquid flow through the connecting tube 11 and prevents the storage solution from flowing into the third product bag 4. The bag set further comprises a supply tube 14 that is connected at one end to the separation bag 1, at the inner edge thereof. The other end of the supply tube 14 is either connected to a cannula, in which case the volume of blood to be separated is to be directly drawn from a donor into the separation bag 1, or connected directly or through a sterile connector 15 to a collection bag 16 connected in turn to a cannula 17 by a donor tube 18 (as shown in figure 1). The bag into which a volume of blood from a donor is to be directly transferred (the separation bag 1 or the collection bag 16) contains a volume of anti-coagulant solution (typically about 70 ml of a solution of sodium citrate for a blood donation of about 450 ml).

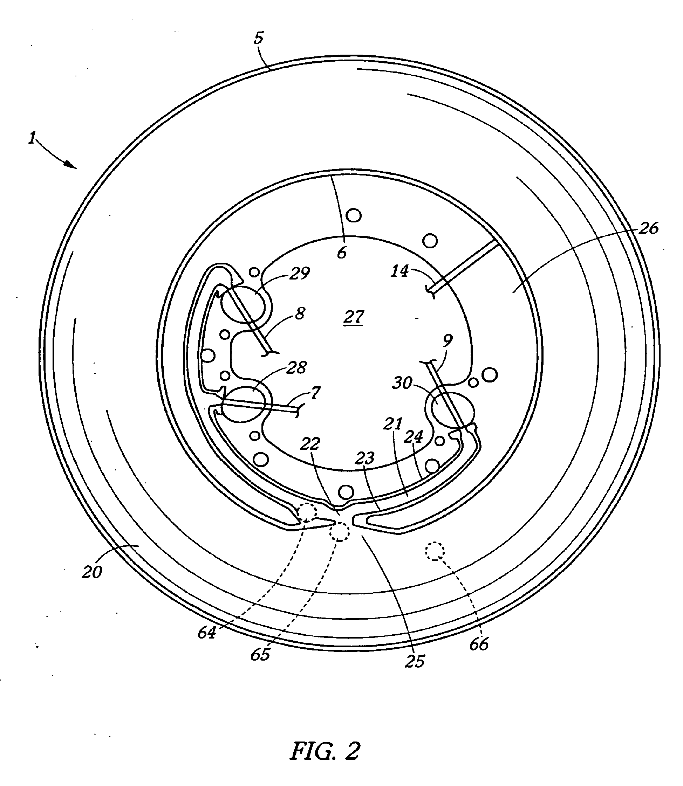

Figure 2 shows a separation bag 1, which is made of two superposed sheets of a flexible plastic material that are joined together by welded lines defining an annular chamber 20 communicating with an inner semi-circular distribution channel 21 via a narrow passage 22. More specifically, the annular chamber 20 is defined by a first circular welded line forming an outer edge 5, and a second circular welded line forming an inner edge 6, the two circular lines being substantially concentric. The distribution channel 21 is defined by two substantially parallel and semi-circular welded lines, forming an outer edge 23 and an inner edge 24 of the distribution channel 21. The inner edge 6 of the annular chamber and the outer edge 24 of the distribution channel 21 join in two points and define therebetween the passage 22. The inner edge 6 of the annular chamber 20 inwardly converges towards both junction points, and the resulting concavity in the otherwise circular inner edge 6 of the annular channel 20 defines a triangular bay area 25 in the annular chamber 20 just upstream of the passage 22.

The passage 22 opens in the distribution channel 21 at about two third of the length of the channel. With respect to the passage 22, the distribution channel 21 can therefore be defined as comprising a longer segment and a smaller segment that are interconnected and extend in opposite directions from the passage 22.The tube 9 connecting the product bag 4 for a red blood cell product to the separation chamber 1 is connected to the smaller segment of the channel 21, at the end thereof. The tube 8 connecting the product bag 3 for a platelet product to the separation chamber 1 is connected to the longer segment of the channel 21, at the end thereof. The tube 7 connecting the product bag 2 for a plasma product to the separation chamber 1 is connected to the longer segment of the channel 21, at about half of its length. The tube 14 for connecting a source of whole blood (donor or collection bag 16) to the separation bag 1 is connected to the annular chamber 1 at the inner edge 6 thereof, at about one third of the circumference of the inner edge 6 from the passage 22, in the same direction as the direction in which the small segment of the distribution channel 21 extends.

The distribution channel 21 and an end portion of the tubes 7, 8, 9, 14 are embedded in a disk-shaped support 26 made of a sheet of semi rigid plastic material, which is secured at its periphery to the inner edge 6 of the annular chamber 20. The disk-shaped support 26 comprises a large cut-out in the middle thereof, as well as three small circular cut-outs 28, 29, 30 adjacent to the connecting points of the tubes 7, 8, 9 to the distribution channel 21. The circular cut-outs 28, 29, 30 are positioned with respect to the end portion of tubes 7, 8, 9 so that each tube extends along a diameter of the corresponding circular cut-out and is therefore maintained straight over a portion of its length by the disk-shaped support 26.

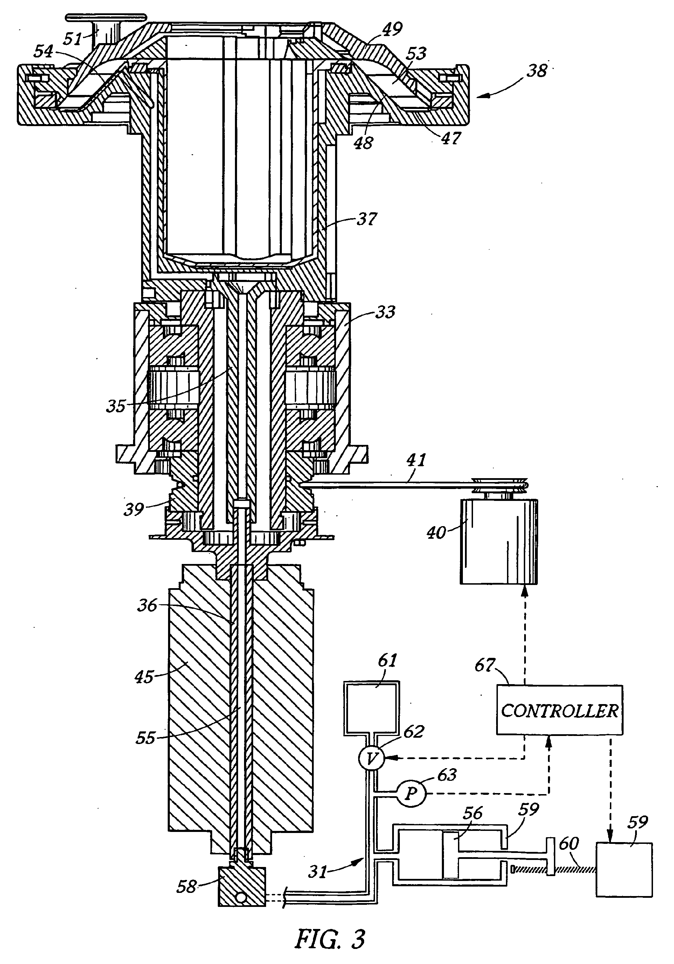

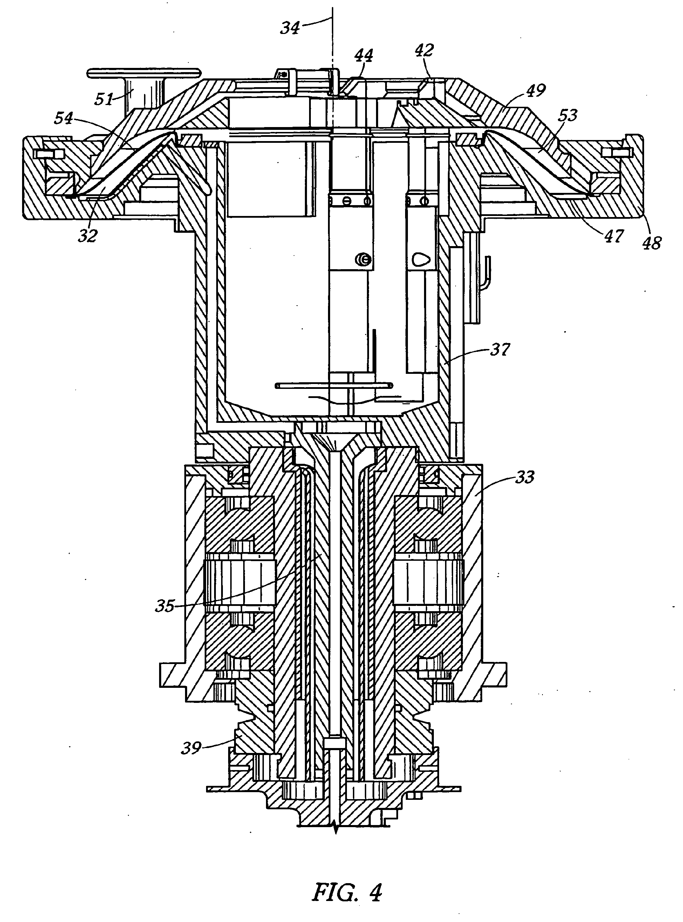

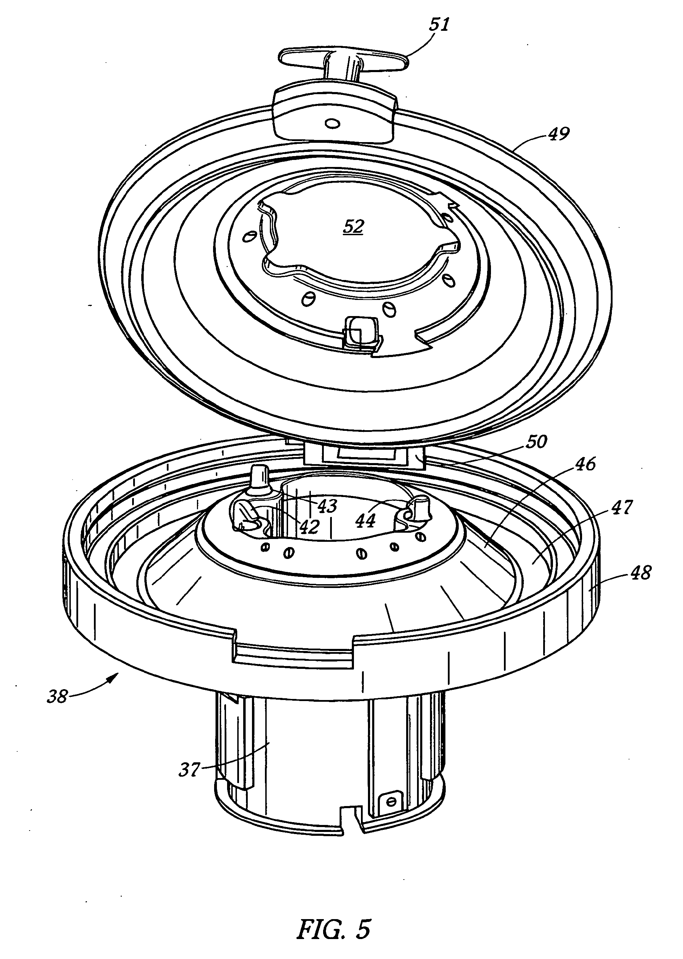

Figures 3, 4, 5 show an apparatus for separating a volume of composite liquid by centrifugation. The apparatus comprises a centrifuge adapted for receiving the set of separation and product bags shown in figures 1 and 2, and a squeezing system for squeezing the separation bag and causing the transfer of separated components into the product bags.

The centrifuge comprises a rotor that is supported by a bearing assembly 33 allowing the rotor to rotate about a vertical central axis 34. The rotor comprises a cylindrical rotor shaft 35, 36, a cylindrical container 37 that is connected to the rotor shaft 35, 36 at the upper end thereof so that the longitudinal axis of the rotor shaft 35, 36 and the longitudinal axis of the container 37 are aligned with the central axis 34 of the rotor, and a circular turntable 38 connected to the container 37 at the upper end thereof so that the central axis of the turntable 37 is aligned with the central axis 34 of the rotor. The rotor shaft comprises a first upper portion 35 and a second lower portion 36. The upper portion 35 of the shaft extends in part through the bearing assembly 33. A pulley 39 is connected to the lower end of the upper portion 35 of the shaft.

The centrifuge further comprises a motor 40 coupled to the rotor by a belt 41 engaged in a groove of the pulley 39 so as to rotate the rotor about the central vertical axis 34.

The separation apparatus further comprises three pinch valve members 42, 43, 44 that are mounted on the rotor for selectively blocking or allowing a flow of liquid through a plastic tube, and selectively sealing and cutting a plastic tube. Each valve 42, 43, 44 comprises an elongated cylindrical body and a head having a groove that is defined by a stationary upper jaw and a lower jaw movable between an open and a closed position, the groove being dimensioned so that one of the plastic tubes 7, 8, 9 of the bag set shown in figures 1 and 2 can be snuggly engaged therein when the lower jaw is in the open position. The elongated body contains a solenoid-actuated mechanism for moving the lower jaw and it is connected to a radio frequency generator providing the energy that is necessary for sealing and cutting a plastic tube. The pinch valve members 42, 43, 44 are mounted inside the cylindrical container 37, adjacent the interior surface thereof, so that their longitudinal axis is parallel to the central axis 34 of the rotor and their heads protrude above the rim of the container 37. The three circular cut-outs 28, 29, 30 of the support portion 26 of the separation bag 1 shown in figure 2 are so dimensioned and positioned as to allow for the engagement of the heads of the three pinch valve members 42, 43, 44 therethrough, with the portions of the tubes 7, 8, 9 extending across the circular cut-outs 28, 29, 30 oriented so as to face the groove in the heads of the pinch valve members 42, 43, 44. Electric power is supplied to the pinch valve members 42, 43, 44 through a slip ring 45 that is mounted around the lower portion 36 of the rotor shaft.

The turntable 38 comprises a central frusto-conical portion 46, the upper, smaller edge of which is connected to the rim of the container 37, an annular flat portion 47 connected to the lower, larger edge of the frusto-conical portion 46 and an outer cylindrical flange 48 extending upwards from the outer periphery of the annular portion 47. The turntable 38 further comprises a vaulted circular lid 49 that is secured to the flange 48 by a hinge 50 so as to pivot between an open and a closed position. The lid 49 is fitted with a lock 51 by which it can be blocked in the closed position. The lid 49 comprises a large cut-out 52 in its upper part that gives access to the cylindrical container 37 of the rotor. The lid 49 has an annular interior surface that is so shaped that, when the lid 49 is in the closed position, it defines with the frusto-conical portion 46 and the annular flat portion 47 of the turntable 38 a frusto-conical annular compartment 53 having a radial cross-section that has substantially the shape of a parallelogram. The frusto-conical annular compartment 53, later the "separation compartment", is intended for containing the separation chamber of the separation bag shown in figure 2.

The squeezing system for squeezing the separation bag within the separation compartment 53 and causing the transfer of separated components into the product bags comprises a flexible annular diaphragm 54 that is so shaped as to line the frusto-conical portion 46 and the annular flat portion 47 of the turntable 38, to which it is secured along its smaller and larger circular edges. The squeezing system further comprises a hydraulic pumping station 31 for pumping a hydraulic liquid in and out an expandable hydraulic chamber 32 defined between the flexible diaphragm 54 and the turntable 38, via a duct 55 extending through the rotor from the lower end of the lower portion 36 of the rotor shaft to the turntable 38. The pumping station 31 comprises a piston pump having a piston 56 movable in a hydraulic cylinder 57 fluidly connected via a rotary fluid coupling 58 to the rotor duct 55. The piston 56 is actuated by a stepper motor 59 that moves a lead screw 60 linked to the piston rod. The hydraulic cylinder 57 is also connected to a hydraulic liquid reservoir 61 having an access controlled by a valve 62 for selectively allowing the introduction or the withdrawal of hydraulic liquid into and from a hydraulic circuit including the hydraulic cylinder 57, the rotor duct 55 and the expandable hydraulic chamber 32. A pressure gauge 63 is connected to the hydraulic circuit for measuring the hydraulic pressure therein.

The separation apparatus further comprises three sensors 64, 65, 66 for detecting characteristics of the separation process occurring within a separation bag when the apparatus operates. The three sensors 64, 65, 66 are embedded in the lid 49 so as to face the separation bag 1 as shown in figure 2, when the lid 49 is closed. The first sensors 64 (later the "channel sensor") is embedded in the lid 49 so as to be positioned over the longer segment of the distribution channel 21. The channel sensor 64 is able to detect the presence of absence of liquid in the distribution channel 21 as well as to detect red blood cells in a liquid. The second sensor 65 (later the "bay sensor") is embedded in the lid 49 so as to be positioned over the bay area 25. The bay sensor 65 is able to detect red blood cells in a liquid. The third sensor 66 (later the "bag sensor") is embedded in the lid 49 so as to be positioned over the separation chamber 20, at about one third of the breadth of the separation chamber from the inner edge 6 of the separation chamber 20, slightly outside of the bay area 25 on the side of the smaller segment of the distribution channel 21. The bag sensor 66 is able to detect red blood cells in a liquid. Each sensor 64, 65, 66 can comprise a photocell including an infra-red LED and a photo-detector.

The separation apparatus further comprises a controller 67 including a microprocessor and a memory for providing the microprocessor with information and programmed instructions relative to the operation of the apparatus. In particular, the microprocessor is programmed for receiving information relative to the various centrifugation speeds at which the rotor is to be rotated during the various stage of a separation process, and information relative to the various transfer flow rates at which separated components are to be transferred from the separation bag 1 into the products bags 2, 3, 4. The information relative to the various transfer flow rates can be expressed, for example, as hydraulic liquid flow rates in the hydraulic circuit, or as rotation speeds of the stepper motor 59 of the hydraulic pumping station 31. The microprocessor is further programmed to receive, directly or through the memory, information from the pressure gauge 63 and from the photocells 64, 65, 66.

The microprocessor is further programmed for controlling the centrifuge motor 40, the stepper motor 59, and the pinch valve members 42, 43, 44 so as to cause the separation apparatus to operate along a predetermined separation protocol.

A first example of separation protocol is as follows.

First stage: the volume of anti-coagulated blood to be separated is transferred into the separation bag before or after the disposable set is loaded in the centrifugation apparatus according to one of the following variants.
- First variant: a volume of anti-coagulated blood to be separated (for example about 500 ml) is transferred into the separation bag before the disposable set is loaded in the centrifugation apparatus. After a clamp has been placed at the connection of each tubes 7, 8, 9 to the separation bag 1, a volume of anti-coagulated blood contained in a collection bag 16 is transferred by gravity in to the separation bag 1. The tube 14 connecting the collection bag 16 to the separation bag is sealed and cut. The separation bag 1 is fitted within the turntable 38, the tubes 7, 8, 9 are engaged in the pinch valve members 42, 44, 43, and the product bags 2, 3, 4, and the secondary bag 10 are placed into the container 37. The pinch valve members 42, 44, 43 are closed and the clamps on the tubes 7, 8, 9 are removed. Alternately, clamps are not placed on tubes 7, 8, 9 when pressure frangible seals are provided in both segments of the distribution channel 21 so as to prevent communication between the product bags 2, 3, 4 and the separation bag 1 as long as the pressure that builds within the separation bag during the operation of the separation apparatus is not high enough to break the frangible seals.
- Second variant: same as the first variant, except that the separation bag 1, which contains a volume of anticoagulant, is directly connected to a donor and the blood of the donor is directly drawn into the separation bag 1, which is thus also used as a collection bag.

Second stage: the air present in the separation bag 1 is purged into the product bag 4 in which the red blood cell component is to be later transferred.

The pinch valve members 42, 43 are closed and the pinch valve member 44 in which the tube 9 is engaged is open. The rotor is set in motion by the centrifuge motor 40 and its rotation speed increases steadily until it rotates at a first, high centrifugation speed (for example, about 3200 RPM). Before the rotor rotates at the first centrifugation speed, the pumping station 31 is actuated so as to pump hydraulic liquid at a constant flow rate (for example, about 240ml/min) into the hydraulic chamber 32 and consequently squeeze the separation bag 1. The air present in the separation bag 1 is expelled into the product bag 4 for the red blood cell component. When the channel sensor 64 detects a liquid in the distribution channel 21, the pumping station 31 is stopped and the pinch valve member 44 is closed.

When the distribution channel 21 of the separation bag 1 is initially closed by frangible seals, the transfer of air from the separation bag 1 into the product bag 4 occurs once the pressure building up in the separation bag 1 is high enough to cause the frangible seals to break.

Note that, alternately, the air contained in the separation bag 1 could be expelled into either the product bag 2 for the plasma component or the product bag 3 for the platelet component. It is however of interest to expel the air in the bag 4 for the red blood cell component because this will allow the red blood cell component to be later transferred by gravity from the product bag 4 into the secondary bag 10.

Third stage: the blood within the separation chamber is sedimented to a desired level.

At the onset of this stage, the three pinch valve members 42, 43 and 44 are closed. The rotor is rotated at the first high centrifugation speed (for example, about 3200 RPM) for a predetermined period of time (for example, about 220 seconds) that is selected so that, whatever the hematocrit of the volume of the blood initially transferred in the separation chamber 1, the blood sediments therein at the end of the selected period to a point where the hematocrit of the outer annular red blood cell layer is about 90 and the inner annular plasma layer plasma does not substantially contain anymore cells, the platelets and the white blood cells occupying then an intermediary annular layer between the red blood cell layer and the plasma layer.

Fourth stage: a first component (plasma component) is transferred into the product bag 2.

At the onset of this stage, the three pinch valve members 42, 43, 44 are closed. Throughout the fourth stage, the rotor is rotated at the first high centrifugation speed (for example, about 3200 RPM). After a predetermined period of time after the bag sensor 66 has stopped detecting red blood cells, which can happen before the end of the predetermined sedimentation period, the pinch valve member 42 controlling the access to the plasma component bag 2 is opened and the pumping station 31 is actuated so as to pump hydraulic liquid at a constant flow rate (for example, about 220ml/min) into the hydraulic chamber 32 and consequently squeeze the separation bag 1 so as to cause the transfer of a first portion of the plasma into the product bag 2, whereas a second portion of the plasma (for example, about 60 ml) remains in the separation bag 1. The pumping station 31 is stopped when a predetermined period of time has lapsed after the bag sensor 66 has detected red blood cells.

The transfer flow rate of the plasma component (which is directly related to the flow rate of the hydraulic fluid) is selected to be as high as possible without disturbing the platelet layer so as to avoid contaminating the plasma component with platelets.

Fifth stage: an Intermediate component (platelet component) is prepared in the separation bag 1.
- First variant: the pinch valve member 43 controlling the access to the platelet component bag 3 is open, and the pinch valve members 42, 44 are closed. The rotation speed of the rotor is rapidly decreased from the first centrifugation speed to a second centrifugation speed (for example, from about 3200 RPM to about 2000 RPM, within about 10 seconds) so as to form an intermediate component resulting from the suspension of the platelets into the second portion of the plasma, whereas the red blood cell layer and the suspended platelet layer remains substantially separated.
- Second variant: the three pinch valve members 42, 43, 44 are closed. The rotation speed of the rotor is rapidly decreased from the first centrifugation speed to a second centrifugation speed (for example, from about 3200 RPM to about 1000 RPM, within about 20 seconds) so as to mix red blood cells, the platelets and the second portion of the plasma. The rotation speed of the rotor is then increased from the second centrifugation speed to a third centrifugation speed, lower that the first centrifugation speed (for example, from about 1000 RPM to about 2500 RPM), so as to separate in the separation bag 1 a red blood cell component and an intermediate component comprising a suspension of platelets in plasma.

Sixth stage: the intermediate component (platelet component) is transferred into the product bag 3.

The pinch valve member 43 controlling the access to the platelet component bag 3 is open and the pinch valve members 42, 44 are closed. The rotor is rotated at the second centrifugation speed (for example, about 2000 RPM, if the preceding stage is the first variant of the fifth stage) or at the third rotation speed (for example, about 2500 RPM, if the preceding stage is the second variant of the fifth stage). The pumping station 31 is actuated so as to pump hydraulic liquid at a first flow rate into the hydraulic chamber 32 and consequently squeeze the separation bag 1 and cause the transfer of the platelet component into the product bag 3. The first flow rate (for example, about 140 ml/min) is substantially lower than the flow rate (for example, about 220 ml/min) at which the plasma component is transferred into the product bag 2 in the fourth stage. The first transfer flow rate of the platelet component (which is directly related to the first flow rate of the hydraulic fluid) is selected to be high enough for preventing the suspended platelets from sedimenting, without at the same time triggering the activation of the platelets.

When the bay sensor 65 detects red blood cells, the pumping station 31 is actuated so as to pump hydraulic liquid into the hydraulic chamber 32 at a second flow rate (for example 40 ml/min) that is substantially lower then the first flow rate, in order to prevent the contamination of the platelet component by red blood cells.

When the hydraulic liquid has been pumped into the hydraulic chamber 32 at the second flow rate for a predetermined period of time (for example, about 4 seconds), the pumping station is actuated so as to pump the hydraulic liquid at a third flow rate (for example, about 20 ml/min) that is lower than the second flow rate, until a predetermined period of time (for example, about 12 seconds) has lapsed after the channel sensor 64 has detected red blood cells. The pumping station 31 is then stopped.

Seventh stage: the third component (red blood cell component) is transferred into the product bag 4.

The pinch valve member 44 controlling the access to the red blood cell component bag 4 is open and the pinch valve members 42, 43 are closed. The rotation speed of the rotor is decreased from the second centrifugation speed (for example, about 2000 RPM) or the third centrifugation speed (for example, about 2500 RPM) to a fourth, lower, centrifugation speed (for example, about 1500 RPM). The pumping station 31 is actuated so as to pump hydraulic liquid at a first flow rate into the hydraulic chamber 32 and consequently squeeze the separation bag 1 so as to cause the transfer of the red blood cell component into the product bag 4. The first flow rate (for example, about 350 ml/min) is substantially higher than the flow rate (for example, about 220 ml/min) at which the plasma component is transferred into the product bag 2 in the fourth stage. The first transfer flow rate of the red blood cell component (which is directly related to the flow rate of the hydraulic fluid) is selected to be as high as possible without damaging the red blood cells (hemolysis).

When the pressure of the hydraulic liquid measured by the pressure gauge 63 reaches a first high pressure threshold (for example, about 0.7 bar), the flow rate of the hydraulic liquid is decreased from the first flow rate to a second flow rate (for example, about 100 ml/min).

When the pressure of the hydraulic liquid measured by the pressure gauge 63 reaches a second high pressure threshold (for example, about 1.6 bar), the flow rate of the hydraulic liquid is further decreased from the second flow rate to a third flow rate (for example, about 37 ml/min).

The second and third transfer flow rates of the red blood cell component (which are directly related to the flow rate of the hydraulic fluid) are selected so that a maximal portion of the red blood cell component is transferred into the red blood cell component bag 4.

When a predetermined period of time (for example, about 30 seconds) has lapsed after the pressure of the hydraulic liquid has reached the second pressure threshold, the rotation speed of the rotor is decreased until the rotor stops, the pumping station 31 is actuated so as to pump the hydraulic liquid from the hydraulic chamber 32 at a high flow rate (for example, about 800 ml/min) until it the hydraulic chamber 32 is empty, and the three pinch valve members 42, 43, 44 are actuated so as to seal and cut the tubes 7, 8, 9.

Any of the at least one first transfer flow rate for the plasma component (one flow rate in the example described above), the at least one second transfer flow rate for the platelet component (three successive flow rates in the example described above), the at least third first transfer flow rate for the red blood cell component (three successive flow rates in the example described above) may be substantially constant, as in the example described above, or it may vary and, for example, comprise a ramp or a series of small steps.

According to a second example of separation protocol, a volume of blood is separated into four components, a plasma component, a first intermediate component (comprising platelets suspended in plasma), a second intermediate component (comprising one of the two types of white blood cells, namely mononuclear cells) and a red blood cell component.

In this second separation protocol, after the stage of transferring the platelet component into a platelet bag, mononuclear cells are transferred into either a specific mononuclear cell bag or into a whole blood collection bag, which, in this case, is not disconnected from the separation bag after the initial stage of transferring the volume of anti-coagulated blood from the collection bag into the separation bag. The transfer of the mononuclear cells occurs at the same rotation speed as the transfer of the platelets (for example, about 2500 RPM), at a transfer flow rate that is higher (for example, about 40ml/mn) than the third and final transfer flow rate of the platelets (for example, about 20ml/mn). The transfer of the mononuclear cell component is completed after a predetermined period of time has lapsed.

According to the second separation protocol, the red blood cells that remain in the separation bag, after the stage of transferring the mononuclear cell component into the mononuclear cell bag, are transferred into a red blood cell bag by gravity, after the centrifuge has stopped and the separation bag has been removed from the centrifuge. In a set of bags adapted to this second protocol, the red blood cell bag is preferably connected at the outer edge of the separation bag. If such a set of bags is used, the air present in the separation bag at the onset of the separation process is then expelled in one of the component bags connected to the inner edge of the separation bag, for example in the mononuclear cell bag.

According to a third example of separation protocol, a volume of blood is separated into three components, a plasma component, an intermediate component (comprising platelets and mononuclear cells), and a red blood cell component.

When the volume of anti-coagulated blood has sedimented to the desired level, then a major portion of the plasma component is transferred into a plasma bag at a maximal flow rate (for example, about 220ml/mn). The transfer of the plasma is stopped after a determined period of time has lapsed after red blood cells are detected by the bag sensor 66 or by the bay sensor 65 depending on the residual volume of plasma that it is desired to leave in the separation bag. The intermediate component (residual plasma, platelets, mononuclear cells and some red blood cells) is then transferred into an intermediate component bag, which can be either a specific component bag or a whole blood collection bag 16. The transfer of the platelet/mononuclear cell component occurs at the same rotation speed as the transfer of the plasma (for example, about 3200 RPM), also at a constant transfer flow rate, but lower (for example, about 140ml/mn) than the transfer flow rate of the plasma. The transfer of the platelet/mononuclear cell component is completed after a pre-determined volume has been expressed into the intermediate component bag.

In the third separation protocol, the red blood cells that remain in the separation bag, after the stage of transferring the platelet/mononuclear cell component into the intermediate component bag, are transferred into a red blood cell bag by gravity, after the centrifuge has stopped and the separation bag has been removed from the centrifuge.

According to a fourth example of separation protocol, a volume of blood is separated into two components, a plasma component and a cell component (platelets, white blood cells and red blood cells). An appropriate set of separation bags may comprise a separation bag connected to a unique component (plasma) bag.

In this fourth separation protocol the air initially present in the separation bag 1 is expelled in the plasma bag. When the volume of anti-coagulated blood has sedimented to the desired level, then a plasma component is transferred into the plasma bag at two different rates: a major portion of the plasma is transferred at a maximal flow rate and the remaining portion of the plasma is then transferred at a lower flow rate. The second flow rate is selected so that a maximum volume of plasma is transferred into the plasma bag and the contamination of the plasma by the blood cells is avoided. The cell component remains in the separation bag for further processing after the centrifuge has stopped.

It will be apparent to those skilled in the art that various modifications can be made to the apparatus and method described herein. Thus, it should be understood that the invention is not limited to the subject matter discussed in the specification. Rather, the present invention is intended to cover modifications and variations.

## Claims

1. A method for separating a volume of a composite liquid into at least a first component and a second component, whereby the volume of composite liquid is contained in a separation bag (1) connected to at least a first component bag (2) and a second component bag (3), the method comprising the steps of:
spinning the separation bag (1) at a first rotation speed so as to centrifuge the volume of composite liquid and cause the sedimentation of the at least first and second components;
when the at least first and second components have sedimented, squeezing the separation bag to transfer at a first transfer flow rate a first portion of the first component into the first component bag (2) during the step of spinning the separation bag at the first rotation speed;
stopping the transfer of the first component before all of the first component has been transferred to the first component bag (2);
rapidly reducing the speed of the centrifuge to a second rotation speed to mix a second, remaining, portion of said first component in the separation bag (1) with a second component; and
squeezing the separation bag (1) to transfer at a second transfer flow rate the mixed first and second components into the second component bag (3), whereby the second transfer flow rate is lower than the first transfer flow rate.

2. A method according to claim 1, wherein the composite liquid is whole blood, the first component comprises plasma and the second component comprises platelets.

3. A method according to claim 1 or 2, wherein the step of squeezing the separation bag to transfer the first portion of the first component comprises:
squeezing the separation bag at a first pressure corresponding to the first transfer flow rate; and
wherein the step of squeezing the separation bag to transfer the mixed first and second components comprises:
squeezing the separation bag at a second pressure less than the first pressure and corresponding to the second transfer flow rate.

4. A method according to claim 1, wherein the method is for separating a volume of the composite liquid into said first component, said second component, and a third component, the spinning step comprising:
spinning the separation bag (1) at said first rotation speed so as to centrifuge the volume of composite liquid and cause the sedimentation of the first, second and third components.

5. A method according to claim 4, wherein the first transfer flow rate is a substantially constant flow rate.

6. A method according to one of claims 4 and 5, wherein the second transfer flow rate comprises an initial flow rate and a final flow rate, the final flow rate being lower than the initial flow rate.

7. A method according to claim 6, wherein the step of transferring into the second component bag (3) comprises the steps of:
transferring into the second component bag (3) at the initial flow rate until the third component is detected at a first location on a pathway of a fluid to the second component bag (3); and
transferring into the second component bag (3) at the final flow rate when the third component is detected at a second location on a pathway of a fluid to the second component bag (3), the first location being upstream of the second location.

8. A method according to any one of the preceding claims, wherein the step of reducing the speed of the centrifuge to mix the second component with the second portion of the first component comprises rapidly decreasing the centrifugation speed from the first rotation speed to the second rotation speed.

9. A method according to any one of claims 4 to 7, wherein the step of reducing the speed of the centrifuge to mix the second component with the second portion of the first component comprises:
rapidly decreasing the centrifugation speed from the first rotation speed to the second rotation speed that is substantially lower than the first rotation speed so as mix the second portion of the first component with the second component and the third component, said method further comprising:
increasing the centrifugation speed from the second rotation speed to a third rotation speed that is lower than the first rotation speed so as to separate the third component from a mix of the second component with the second portion of the first component.

10. A method according to any one of claims 4 to 9, further comprising the step of transferring the third component from the separation bag (1) into a third component bag (4) connected to the separation bag (1) at a third, transfer flow rate, whereby the third transfer rate is different from the second transfer flow rate.

11. A method according to claim 10, wherein the third transfer flow rate comprises an initial flow rate and a final flow rate, the final flow rate being lower than the initial flow rate.

12. A method according to any one of claims 10 or 11, further comprising spinning the separation bag (1) during the transfer of the third component from the separation bag (1) into the third component bag (4) at a third rotation speed that is less than the second rotation speed.

13. A method according to any one of claims 10 to 12, wherein the step of transferring the first portion of the first component from the separation bag (1) into the first component bag (2) comprises:
allowing a flow of fluid through a first tube (7) connecting the separation bag (1) to the first component bag (2);
blocking a flow of fluid through a second tube (8) connecting the separation bag (1) to the second component bag (3);
blocking a flow of fluid through a third tube (9) connecting the separation bag (1) to the third component bag (4); and
squeezing the separation bag (1) until the third component is detected on a pathway of a fluid to the first component bag (2).

14. A method according to any one of claims 10 to 13, wherein the step of transferring into the second component bag (3) comprises:
blocking a flow of fluid through a first tube (7) connecting the separation bag (1) to the first component bag (2);
allowing a flow of fluid through a second tube connecting the separation bag (1) to the second component bag (3);
blocking a flow of fluid through a third tube (9) connecting the separation bag (1) to the third component bag (4); and
squeezing the separation bag (1) until the third component is detected on a pathway of a fluid to the second component bag (3).

15. A method according to any one of claims 10 to 14, wherein the step of transferring the third component from the separation bag (1) into the third component bag (4) comprises:
blocking a flow of fluid through a first tube (7) connecting the separation bag (1) to the first component bag (2);
blocking a flow of fluid through a second tube (8) connecting the separation bag (1) to the second component bag (3);
allowing a flow of fluid through a third tube (9) connecting the separation bag (1) to the third component bag (4); and
squeezing the separation bag (1) until it is substantially empty.

16. A method according to claim 15, further comprising the steps of:
detecting when the separation bag (1) is substantially empty, and
stopping spinning the separation bag (1) after detecting that the separation bag (1) is substantially empty.

17. A method according to any one of claims 10 to 16, wherein the step of squeezing the separation bag (1) comprises:
submitting the separation bag to a hydraulic pressure; and
measuring the hydraulic pressure, and
wherein the step of transferring the third component from the separation bag (1) into the third component bag (4) comprises:
transferring the third component at a third flow rate until the measured hydraulic pressure reaches a determined pressure threshold; and
transferring the third component at a fourth flow rate after the measured hydraulic pressure has reached the determined pressure threshold, the fourth flow rate being lower than the third flow rate.

18. A method according to any one of claims 10 to 17, further comprising the step of transferring air from the separation bag (1) into the third component bag (4) before transferring the first portion of the first component from the separation bag (1) into the first component bag (2), said step comprising:
blocking a flow of fluid through a first tube (7) connecting the separation bag (1) to the first component bag (2);
blocking a flow of fluid through a second tube (8) connecting the separation bag (1) to the second component bag (3);
allowing a flow of fluid through a third tube (9) connecting the separation bag (1) to the third component bag (4); and
squeezing the separation bag (1) until a liquid is detected on a pathway of fluid to the third component bag (4).

19. A method according to any one of claims 4 to 18, wherein the composite liquid comprises whole blood, the first component comprises plasma, the second component comprises platelets and the third component comprises red blood cells.

20. A method according to one of the preceding claims, further comprising the step of transferring air from the separation bag (1) into one of the component bags before transferring the first portion of the first component from the separation bag (1) into the first component bag (2).

21. A method according one of the preceding claims, wherein any step of squeezing the separation bag (1) comprises submitting the separation bag (1) to a hydraulic pressure.

22. A method according to claim 1, wherein the composite liquid comprises whole blood, the first component comprises plasma and the second component comprises platelets, white blood cells and red blood cells.

## Patentansprüche

1. Verfahren zur Trennung eines Volumens einer zusammengesetzten Flüssigkeit in zumindest einen ersten Bestandteil und einen zweiten Bestandteil, wobei das Volumen der zusammengesetzten Flüssigkeit in einem Separationsbeutel (1) enthalten ist, der an einen Beutel eines ersten Bestandteils (2) angeschlossen ist, wobei das Verfahren folgende Schritte enthält:
Schleudern des Separationsbeutels (1) bei einer ersten Rotationsgeschwindigkeit, um so das Volumen der zusammengesetzten Flüssigkeit zu zentrifugieren und die Sedimentation von den zumindest ersten und zweiten Bestandteilen zu veranlassen;
wenn die zumindest ersten und zweiten Bestandteile sedimentiert sind, wird der Separationsbeutel gepresst, um bei einer ersten Übertragungsflussrate einen ersten Teil des ersten Bestandteiles in den Beutel des ersten Bestandteils (2) während des Schrittes zum Schleudern des Separationsbeutels bei der ersten Rotationsgeschwindigkeit zu übertragen;
die Übertragung des ersten Bestandteils wird beendet, bevor der gesamte erste Bestandteil in den Beutel des ersten Bestandteils (2) übertragen worden ist;
die Geschwindigkeit der Zentrifuge wird schnell auf eine zweite Rotationsgeschwindigkeit verringert, um einen zweiten verbliebenen Teil des ersten Bestandteils in dem Separationsbeutel (1) mit einem zweiten Bestandteil zu vermischen; und
der Separationsbeutel (1) wird gepresst, um bei einer zweiten Übertragungsflussrate die gemischten ersten und zweiten Bestandteile in den Beutel des zweiten Bestandteils (3) zu übertragen, wobei die zweite Übertragungsflussrate niedriger als die erste Übertragungsflussrate ist.

2. Verfahren nach Anspruch 1, wobei die zusammengesetzte Flüssigkeit Gesamtblut ist, der erste Bestandteil Plasma aufweist und der zweite Bestandteil Blutplättchen aufweist.

3. Verfahren nach einem der Ansprüche 1 oder 2, wobei der Schritt zum Pressen des Separationsbeutels zum Übertragen des ersten Teils des ersten Bestandteils aufweist:
Pressen des Separationsbeutels bei einem ersten Druck entsprechend zu der ersten Übertragungsflussrate; und
wobei der Schritt zum Pressen des Separationsbeutels zum Übertragen der gemischten ersten und zweiten Bestandteile aufweist:
Pressen des Separationsbeutels bei einem zweiten Druck, der geringer als der erste Druck ist, und entsprechend zu der zweiten Übertragungsflussrate.

4. Verfahren nach Anspruch 1, wobei das Verfahren zum Separieren eines Volumens der zusammengesetzten Flüssigkeit in den ersten Bestandteil, den zweiten Bestandteil und einen dritten Bestandteil den Schritt zum Schleudern aufweist:
Schleudern des Separationsbeutels (1) bei der ersten Rotationsgeschwindigkeit, um so das Volumen der zusammengesetzten Flüssigkeit zu zentrifugieren und die Sedimentation der ersten, zweiten und dritten Bestandteile zu verursachen.

5. Verfahren nach Anspruch 4, wobei die erste Übertragungsflussrate eine im Wesentlichen konstante Flussrate ist,

6. Verfahren nach einem der Ansprüche 4 oder 5, wobei die zweite Übertragungsflussrate eine anfängliche Flussrate und eine schließliche Flussrate aufweist, wobei die schließliche Flussrate geringer als die anfängliche Flussrate ist.

7. Verfahren nach Anspruch 6, wobei der Schritt zum Übertragen in den Beutel des zweiten Bestandteils (3) die folgenden Schritte aufweist:
in den Beutel des zweiten Bestandteils (3) wird bei der anfänglichen Flussrate übertragen bis der dritte Bestandteil an einem ersten Ort auf einem Zuweg von einem Fluid zu dem Beutel des zweiten Bestandteils (3) detektiert wird; und
in den Beutel des zweiten Bestandteils (3) wird bei der schließlichen Flussrate hubertragen, wenn der dritte Bestandteil an einem zweiten Ort auf dem Zuweg eines Fluids zu dem Beutel des zweiten Bestandteils (3) detektiert wird, wobei der erste Ort stromaufwärts des zweiten Ortes ist.

8. Verfahren nach irgendeinem der voranstehenden Ansprüche, wobei der Schritt zum Verringern der Geschwindigkeit der Zentrifuge zum Mischen des zweiten Bestandteils mit dem zweiten Teil der ersten Bestandteils ein schnelles Verringern der Zentrifugalgeschwindigkeit von der ersten Rotationsgeschwindigkeit zu der zweiten Rotationsgeschwindigkeit aufweist.

9. Verfahren nach irgendeinem der Ansprüche 4 bis 7, wobei der Schritt zum Verringern der Geschwindigkeit der Zentrifuge zum Mischen des zweiten Bestandteils mit dem zweiten Teil des ersten Bestandteils aufweist:
schnelles Verringern der Zentrifugalgeschwindigkeit von der ersten Rotationsgeschwindigkeit zu der zweiten Rotationsgeschwindigkeit, die im wesentlichen niedriger als die erste Rotationsgeschwindigkeit ist, um so den zweiten Teil des ersten Bestandteils mit dem zweiten Bestandteil und dem dritten Bestandteil zu mischen, wobei das Verfahren aufweist:
Erhöhen der Zentrifugalgeschwindigkeit von der zweiten Rotationsgeschwindigkeit zu einer dritten Rotationsgeschwindigkeit, die niedriger als die erste Rotationsgeschwindigkeit ist, um so den dritten Bestandteil von einer Mischung des zweiten Bestandteils mit dem zweiten Teil des ersten Bestandteils zu trennen.

10. Verfahren nach irgendeinem der Ansprüche 4 bis 9, das ferner den Schritt zum Übertragen des dritten Bestandteils von dem Separationsbeutel (1) in einen Beutel eines dritten Bestandteils (4), der an den Separationsbeutel (1) angeschlossen ist, bei einer dritten Übertragungsflussrate aufweist, wobei die dritte Übertragungsrate verschieden von der zweiten Übertragungsflussrate ist.

11. Verfahren nach Anspruch 10, wobei die dritte Übertragungsflussrate aufweist, eine anfängliche Flussrate und eine schließliche Flussrate, wobei die schließliche Flussrate niedriger als die anfängliche Flussrate ist.

12. Verfahren nach irgendeinem der Ansprüche 10 oder 11, das aufweist, Schleudern des Separationsbeutels (1) während der Übertragung des dritten Bestandteils von dem Separationsbeutel (1) in den Beutel des dritten Bestandteils (4) bei einer dritten Rotationsgeschwindigkeit, die geringer als die zweite Rotationsgeschwindigkeit ist.

13. Verfahren nach irgendeinem der Ansprüche 10 bis 12, wobei der Schritt zum Übertragen des ersten Teils des ersten Bestandteils von dem Separationsbeutel (1) in den Beutel des ersten Bestandteils (2) aufweist:
ein Fluss eines Fluids durch ein erstes Rohr (7), das den Separationsbeutel (1) mit dem Beutel des ersten Bestandteils (2) verbindet, wird ermöglicht;
ein Strom eines Fluids durch ein zweites Rohr (8), das den Separationsbeutel (1) mit dem Beutel des zweiten Bestandteils (3) verbindet, wird blockiert;
ein Strom eines Fluids durch ein drittes Rohr (9), das den Separationsbeutel (1) mit dem Beutel des dritten Bestandteils (4) verbindet, wird blockiert; und
der Separationsbeutel (1) wird gepresst bis der dritte Bestandteil auf einem Zuweg eines Fluids zu dem Beutel des ersten Bestandteils (2) detektiert wird.

14. Verfahren nach irgendeinem der Ansprüche 10 bis 13, wobei der Schritt zum Übertragen in den Beutel des zweiten Bestandteils (3) aufweist:
ein Strom eines Fluids durch ein erstes Rohr (7), das den Separationsbeutel (1) an den Beutel des ersten Bestandteils (2) anschließt, wird blockiert;
ein Strom eines Fluids durch ein zweites Rohr, das den Separationsbeutel (1) an den Beutel des dritten Bestandteils (4) anschließt, wird blockiert, und
der Separationsbeutel (1) wird gepresst bis der dritte Bestandteil auf einem Zuweg eines Fluids zu dem Beutel des ersten Bestandteils (2) detektiert wird.

15. Verfahren nach irgendeinem der Ansprüche 10 bis 14, wobei der Schritt zum Übertragen des dritten Bestandteils von dem Separationsbeutel (1) zu dem Beutel des dritten Bestandteils (4) aufweist:
ein Strom eines Fluids durch ein erstes Rohr (7), das den Separationsbeutel (1) an den Beutel des ersten Bestandteils (2) abschließt, wird blockiert;
ein Strom eines Fluids durch ein zweites Rohr (8), das den Separationsbeutel (1) an den Beutel des zweiten Bestandteils (3) anschließt, wird blockiert;
ein Strom eines Fluids durch ein drittes Rohr (9), das den Separationsbeutel (1) an den Beutel des dritten Bestandteils (4) anschließt, wird ermöglicht; und
der Separationsbeutel (1) wird gepresst, bis er im Wesentlichen leer ist.

16. Verfahren nach Anspruch 15, das ferner die Schritte aufweist:
es wird detektiert, wann der Separationsbeutel (1) im Wesentlichen leer ist, und
das Schleudern des Separationsbeutels (1) wird angehalten, nachdem detektiert wort den ist, dass der Separationsbeutel (1) im Wesentlichen leer ist.

17. Verfahren nach irgendeinem der Ansprüche 10 bis 16, wobei der Schritt zum Pressen des Separationsbeutels (1) aufweist:
der Separationsbeutel wird einem hydraulischen Druck ausgesetzt; und
der hydraulische Druck wird gemessen, und
wobei der Schritt zum Übertragen des dritten Bestandteils von dem Separationsbeutel (1) in den Beutel des dritten Bestandteils (4) aufweist:
der dritte Bestandteil wird bei einer dritten Flussrate übertragen, bis der gemessene hydraulische Druck einen bestimmten Druckschwellwert erreicht; und
der dritte Bestandteil wird bei einer vierten Flussrate übertragen, nachdem der gemessene hydraulische Druck den vorbestimmten Druckschwellwert erreicht hat, wobei die vierte Flussrate niedriger als die dritte Flussrate ist.

18. Verfahren nach irgendeinem der Ansprüche 10 bis 17, das ferner den Schritt zum Übertragen von Luft von dem Separationsbeutel (1) in den Beutel des dritten Bestandteils (4) aufweist, bevor der erste Teil des ersten Bestandteils von dem Separationsbeutel (1) in den Beutel des ersten Bestandteils (2) übertragen wird, wobei der Schritt aufweist:
ein Strom eines Fluids durch ein erstes Rohr (7), das den Separationsbeutel (1) an den Beutel des ersten Bestandteils (2) anschließt, wird blockiert;
ein Strom eines Fluids durch ein zweites Rohr (8), das den Separationsbeutel (1) an den Beutel des zweiten Bestandteils (3) anschließt, wird blockiert;
ein Strom eines Fluids durch ein drittes Rohr (9), das den Separationsbeutel (1) an den Beutel des dritten Bestandteils (4) anschließt, wird ermöglicht; und
der Separationsbeutel (1) wird gepresst bzw. gequetscht, bis eine Flüssigkeit auf einem Zuweg von Fluid zu dem Beutel des dritten Bestandteils (4) detektiert wird.

19. Verfahren nach irgendeinem der Ansprüche 4 bis 18, wobei die zusammengesetzte Flüssigkeit aufweist, Gesamtblut, den ersten Bestandteil, der Plasma aufweist, den zweiten Bestandteil, der Blutplättchen aufweist, und den dritten Bestandteil, der rote Blutzellen aufweist.

20. Verfahren nach irgendeinem der voranstehenden Ansprüche, das ferner den Schritt zum Übertragen von Luft von dem Separationsbeutel (1) in einen der Beutel der Bestandteile aufweist, bevor der erste Teil des ersten Bestandteils von dem Separationsbeutel (1) in den Beutel des ersten Bestandteils (2) übertragen wird.

21. Verfahren nach irgendeinem der voranstehenden Ansprüche, wobei irgendein Schritt zum Pressen bzw. Quetschen des Separationsbeutels (1) aufweist, dass der Separationsbeutel (1) einem hydraulischen Druck ausgesetzt wird.

22. Verfahren nach Anspruch 1, wobei die zusammengesetzte bzw. -gemischte Flüssigkeit aufweist, Gesamtblut, den ersten Bestandteil, der Plasma aufweist, und den zweiten Bestandteil, der Blutplättchen, weiße Blutzellen und rote Blutzellen aufweist.

## Revendications

1. Procédé de séparation d'un volume d'un liquide composite en au moins un premier composant et un deuxième composant, dans lequel le volume du liquide composite est contenu dans une poche de séparation (1) reliée à au moins une première poche de composant (2) et au moins une deuxième poche de composant (3), le procédé comprenant les étapes de :
mise en rotation de la poche de séparation (1) à une première vitesse de rotation de manière à centrifuger le volume du liquide composite et provoquer la sédimentation des au moins premier et deuxième composants ;
lorsque les au moins premier et deuxième composants se sont déposés, compression de la poche de séparation pour transférer à un premier débit de transfert une première partie du premier composant dans la première poche de composant (2) pendant l'étape de mise en rotation de la poche de séparation à la première vitesse de rotation ;
arrêt du transfert du premier composant avant que l'ensemble du premier composant ait été transféré vers la première poche de composant (2) ;
réduction rapide de la vitesse de la centrifugeuse à une deuxième vitesse de rotation pour mélanger une seconde partie résiduelle dudit premier composant dans la poche de séparation (1) avec un deuxième composant ; et
compression de la poche de séparation (1) pour transférer à un deuxième débit de transfert les premier et deuxième composants mélangés dans la deuxième poche de composant (3), le deuxième débit de transfert étant plus faible que le premier débit de transfert.

2. Procédé selon la revendication 1, dans lequel le liquide composite est du sang total, le premier composant comprend du plasma et le deuxième composant comprend des plaquettes.

3. Procédé selon la revendication 1 ou 2, dans lequel l'étape de compression de la poche de séparation pour transférer la première partie du premier composant comprend :
la compression de la poche de séparation à une première pression correspondant au premier débit de transfert ; et
l'étape de compression de la poche de séparation pour transférer les premier et deuxième composants mélangés comprend :
la compression de la poche de séparation à une seconde pression inférieure à la première pression et correspondant au deuxième débit de transfert.

4. Procédé selon la revendication 1, dans lequel le procédé est destiné à séparer un volume du liquide composite en ledit premier composant, ledit deuxième composant et un troisième composant, l'étape de mise en rotation comprenant :
la mise en rotation de la poche de séparation (1) à ladite première vitesse de rotation de manière à centrifuger le volume du liquide composite et à provoquer la sédimentation du premier, du deuxième et du troisième composants.

5. Procédé selon la revendication 4, dans lequel le premier débit de transfert est un débit sensiblement constant.

6. Procédé selon l'une des revendications 4 et 5, dans lequel le deuxième débit de transfert comprend un débit initial et un débit final, le débit final étant plus faible que le débit initial.

7. Procédé selon la revendication 6, dans lequel l'étape de transfert dans la deuxième poche de composant (3) comprend les étapes de :
transfert dans la deuxième poche de composant (3) au débit initial jusqu'à ce que le troisième composant soit détecté à un premier emplacement sur la trajectoire d'un fluide vers la deuxième poche de composant (3) ; et
transfert dans la deuxième poche de composant (3) au débit final lorsque le troisième composant est détecté à un second emplacement sur la trajectoire d'un fluide vers la deuxième poche de composant (3), le premier emplacement étant en amont du second emplacement.

8. Procédé selon l'une quelconque des revendications précédentes, dans lequel l'étape de réduction de la vitesse de la centrifugeuse pour mélanger le deuxième composant avec la seconde partie du premier composant comprend la réduction rapide de la vitesse de centrifugation depuis la première vitesse de rotation à la deuxième vitesse de rotation.

9. Procédé selon l'une quelconque des revendications 4 à 7, dans lequel l'étape de réduction de la vitesse de la centrifugeuse pour mélanger le deuxième composant avec la seconde partie du premier composant comprend :
la réduction rapide de la vitesse de centrifugation depuis la première vitesse de rotation à la deuxième vitesse de rotation qui est sensiblement inférieure à la première vitesse de rotation de manière à mélanger la seconde partie du premier composant avec le deuxième composant et le troisième composant, ledit procédé comprenant en outre :
l'augmentation de la vitesse de centrifugation depuis la deuxième vitesse de rotation à la troisième vitesse de rotation qui est inférieure à la première vitesse de rotation de manière à séparer le troisième composant d'un mélange du deuxième composant avec la seconde partie du premier composant.

10. Procédé selon l'une quelconque des revendications 4 à 9, comprenant en outre l'étape de transfert du troisième composant depuis la première poche de séparation (1) dans une troisième poche de composant (4) reliée à la poche de séparation (1) à un troisième débit de transfert, le troisième débit de transfert étant différent du deuxième débit de transfert.

11. Procédé selon la revendication 10, dans lequel le troisième débit de transfert comprend un débit initial et un débit final, le débit final étant plsu faible que le débit initial.

12. Procédé selon l'une quelconque des revendications 10 et 11, comprenant en outre la mise en rotation de la poche de séparation (1) pendant le transfert du troisième composant depuis la poche de séparation (1) dans la troisième poche de composant (4) à une troisième vitesse de rotation qui est inférieure à la deuxième vitesse de rotation.

13. Procédé selon l'une quelconque des revendications 10 à 12, dans lequel l'étape de transfert de la première partie du premier composant de la poche de séparation (1) dans la première poche de composant (2) comprend :
la permission d'un écoulement de fluide à travers un premier tube (7) reliant la poche de séparation (1) à la première poche de composant (2) ;
le blocage d'un écoulement de fluide à travers un deuxième tube (8) reliant la poche de séparation (1) à la deuxième poche de composant (3) ;
le blocage d'un écoulement de fluide à travers un troisième tube (9) reliant la poche de séparation (1) à la troisième poche de composant (4) ; et
la compression de la poche de séparation (1) jusqu'à ce que le troisième composant soit détecté sur une trajectoire d'un fluide vers la première poche de composant (2).

14. Procédé selon l'une quelconque des revendications 10 à 13, dans lequel l'étape de transfert dans la deuxième poche de composant (3) comprend :
le blocage d'un écoulement de fluide à travers un premier tube (7) reliant la poche de séparation (1) à la première poche de composant (2) ;
la permission d'un écoulement de fluide à travers un deuxième tube reliant la poche de séparation (1) à la deuxième poche de composant (3) ;
le blocage d'un écoulement de fluide à travers un troisième tube (9) reliant la poche de séparation (1) à la troisième poche de composant (4) ; et
la compression de la poche de séparation (1) jusqu'à ce que le troisième composant soit détecté sur une trajectoire d'un fluide vers la deuxième poche de composant (3).

15. Procédé selon l'une quelconque des revendications 10 à 14, dans lequel l'étape de transfert du troisième composant de la poche de séparation (1) dans la troisième poche de composant (4) comprend :
le blocage d'un débit de fluide à travers un premier tube (7) reliant la poche de séparation (1) à la première poche de composant (2) ;
le blocage d'un écoulement de fluide à travers un deuxième tube (8) reliant la poche de séparation (1) à la deuxième poche de composant (3) ;
la permission d'un écoulement de fluide à travers un troisième tube (9) reliant la poche de séparation (1) à la troisième poche de composant (4) ; et
la compression de la poche de séparation (1) jusqu'à ce qu'elle soit sensiblement vide.

16. Procédé selon la revendication 15, comprenant en outre les étapes de :
détection du moment où la poche de séparation (1) est sensiblement vide, et
arrêt de la mise en rotation de la poche de séparation (1) après avoir détecté que la poche de séparation (1) est sensiblement vide.

17. Procédé selon l'une quelconque des revendications 10 à 16, dans lequel l'étape de compression de la poche de séparation (1) comprend :
la soumission de la poche de séparation à une pression hydraulique ; et
la mesure de la pression hydraulique, et
dans lequel l'étape de transfert du troisième composant depuis la poche de séparation (1) dans la troisième poche de composant (4) comprend :
le transfert du troisième composant à un troisième débit jusqu'à ce que la pression hydraulique mesurée atteigne un seuil de pression déterminé ; et
le transfert du troisième composant à un quatrième débit une fois que la pression hydraulique mesurée a atteint le seuil de pression déterminé, le quatrième débit étant plus faible que le troisième débit.

18. Procédé selon l'une quelconque des revendications 10 à 17, comprenant en outre l'étape de transfert de l'air provenant de la poche de séparation (1) dans la troisième poche de composant (4) avant de transférer la première partie du premier composant depuis la poche de séparation (1) dans la première poche de composant (2), ladite étape comprenant :
le blocage d'un écoulement de fluide à travers un premier tube (7) reliant la poche de séparation (1) à la première poche de composant (2) ;
le blocage d'un écoulement de fluide à travers un deuxième tube (8) reliant la poche de séparation (1) à la deuxième poche de composant (3) ;
la permission d'un écoulement de fluide à travers un troisième tube (9) reliant la poche de séparation (1) à la troisième poche de composant (4) ; et
la compression de la poche de séparation (1) jusqu'à ce qu'un liquide soit détecté sur une trajectoire du fluide vers la troisième poche de composant (4).

19. Procédé selon l'une quelconque des revendications 4 à 18, dans lequel le liquide composite comprend du sang total, le premier composant comprend du plasma, le deuxième composant comprend des plaquettes et le troisième composant comprend des globules rouges.

20. Procédé selon une des revendications précédentes, comprenant en outre l'étape de transfert de l'air depuis la poche de séparation (1) dans une des poches de composant avant de transférer la première partie du premier composant de la poche de séparation (1) dans la première poche de composant (2).

21. Procédé selon une des revendications précédentes, dans lequel toute étape de compression de la poche de séparation (1) comprend la soumission de la poche de séparation (1) à une pression hydraulique.

22. Procédé selon la revendication 1, dans lequel le liquide composite comprend du sang total, le premier composant comprend du plasma et le deuxième composant comprend des plaquettes, des globules blancs et des globules rouges.
